(19) Europäisches Patentamt
European Patent Office
Office européen des brevets

(11) Veröffentlichungsnummer: **0 421 264 B1**

(12) **EUROPÄISCHE PATENTSCHRIFT**

(45) Veröffentlichungstag der Patentschrift: **10.08.94**

(51) Int. Cl.[5]: **C08F 20/04**, C08F 2/08, A61L 15/60, A61L 15/24

(21) Anmeldenummer: **90118525.6**

(22) Anmeldetag: **27.09.90**

# (54) Hydrophile, quellbare Polymerisate.

(30) Priorität: **06.10.89 DE 3933351**

(43) Veröffentlichungstag der Anmeldung:
**10.04.91 Patentblatt 91/15**

(45) Bekanntmachung des Hinweises auf die Patenterteilung:
**10.08.94 Patentblatt 94/32**

(84) Benannte Vertragsstaaten:
**AT BE CH DE DK ES FR GB IT LI NL SE**

(56) Entgegenhaltungen:
**FR-A- 1 082 268**
**US-A- 4 286 082**

(73) Patentinhaber: **CASSELLA Aktiengesellschaft**
**Hanauer Landstrasse 526**
**D-60386 Frankfurt(DE)**

(72) Erfinder: **Engelhardt, Fritz, Dr.**
**Hünfelder Strasse 20**
**W-6000 Frankfurt am Main 61(DE)**
Erfinder: **Ebert, Gerlinde, Dr.**
**Am Tannenstumpf 26**
**W-6072 Dreieicht/Offenthal(DE)**

(74) Vertreter: **Urbach, Hans-Georg, Dr. et al**
**Hanauer Landstrasse 526**
**D-60386 Frankfurt (DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft hydrophile, quellbare Polymerisate, ihre Herstellung und Verwendung.

Quellbare Polymere, die wäßrige Lösungen absorbieren, werden für die Herstellung von Tampons, Windeln, Damenbinden und anderen Hygieneartikeln sowie als wasserzurückhaltende Mittel im landwirtschaftlichen Gartenbau verwendet.

Zu bekannten Absorptionsharzen dieses Typs gehören vernetzte Carboxymethylcellulose, teilweise vernetztes Polyalkylenoxid, Hydrolysate von Stärke-Acrylnitril-Pfropfcopolymeren oder teilweise vernetzte Polyacrylsäuresalze.

Diese bekannten Polymerisate zeigen durchweg Nachteile, insbesondere bei der Absorption wäßriger Elektrolytlösung sowie Blut und Urin.

Bei hohem Absorptionsvermögen werden nach dem derzeitigen Stand der Technik zu geringe Gelfestigkeiten der gequollenen Polymerpartikel erreicht. Es bilden sich klebrige Massen, welche die Saugfähigkeit der damit hergestellten Produkte verschlechtern.

Es ist bekannt, daß durch Erhöhung der Vernetzungsdichte die Gelfestigkeit sowie die Geschwindigkeit der Flüssigkeitsaufnahme erhöht werden kann, dadurch jedoch gleichzeitig die Absorptionskapazität herabgesetzt wird. Diese Vorgehensweise ist insofern unerwünscht als die Absorptionskapazität die wichtigste Eigenschaft des Polymeren ist.

Aufgabe der vorliegenden Erfindung ist es, Polymere, die wäßrige Lösungen absorbieren, bereitzustellen, welche eine hohe Absorptionsrate aufweisen und dabei im gequollenen Zustand nicht klebende Hydrogelpartikel hoher Gelfestigkeit bilden.

Diese Aufgabe wird in überraschender Weise gelöst durch hydrophile, quellbare Polymerisate, die in statistischer Verteilung zu 98 bis 100 Gew.% aus Resten der allgemeinen Formel I

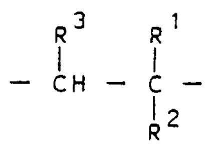 (I)

worin

$R^1$ Wasserstoff, Methyl oder Ethyl

$R^2$ Carboxyl, Sulfonyl, Phosphonyl, die gegebenenfalls mit Alkanol mit 1 bis 4 Kohlenstoffatomen verestert sein können; Phenyl; Sulfonylphenyl; Pyrrolidonyl; Pyridyl; Imidazolyl; eine Gruppe der Formel

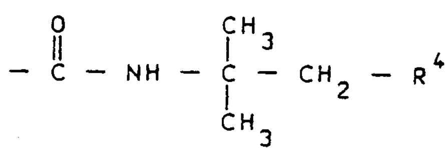

worin $R^4$ für die Sulfonyl- oder die Phosphonylgruppe steht; Cyan; Chlor; die $-CONH_2$-Gruppe; $(C_1-C_4)$-Alkanoyloxy; oder eine Gruppe der Formel

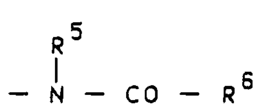

worin $R^5$ und $R^6$ unabhängig voneinander Wasserstoff, Methyl oder Ethyl bedeuten oder gegebenenfalls auch zusammen für Trimethylen Stehen; und

$R^3$ Wasserstoff, Methyl, Ethyl oder Carboxy bedeuten, wobei saure Gruppen auch in Salzform vorliegen können, und zu 0 - 2 Gew.% aus Resten eines Vernetzers, die aus Monomeren mit mindestens zwei olefinisch ungesättigten Doppelbindungen hervorgegangen sind, bestehen, und dadurch gekennzeichnet sind, daß sie in Form einer hochporösen, schaumartigen Polyederstruktur vorliegen.

Die hochporöse, schaumartige Polyederstruktur weist einen mittleren Porendurchmesser von 0,8 bis 1,2 mm auf.

Die bevorzugte Dichte der erfindungsgemäßen Polymerisate liegt zwischen 0,01 und 0,05 g/cm³.

Bevorzugte erfindungsgemäße Polymerisate bestehen zu 98,5 bis 99,7 Gew.% aus Resten der allgemeinen Formel I und zu 0,3 bis 1,5 Gew.% aus vernetzenden Strukturen.

In den erfindungsgemäßen Polymerisaten können die Reste der allgemeinen Formel I alle exakt die gleiche Struktur haben, sie können sich aber auch hinsichtlich der Reste $R^1$, $R^2$ und $R^3$ voneinander unterscheiden. Im letztgenannten Fall können bezüglich der Bedeutungen von $R^1$, $R^2$ und $R^3$ verschiedene Reste der allgemeinen Formel I in statistischer Weise abwechseln, es können aber auch größere Polymerabschnitte aufeinander folgen, in denen $R^1$, $R^2$ und $R^3$ jeweils nur eine Bedeutung haben.

In den Resten der allgemeinen Formel I bedeutet

$R^1$ bevorzugt Wasserstoff oder Methyl.

$R^2$ steht bevorzugt für Carboxyl, Sulfonyl oder Phosphonyl, wobei Carboxyl besonders bevorzugt ist.

$R^3$ bedeutet bevorzugt Wasserstoff.

In den Resten der allgemeinen Formel I können die sauren Gruppen ganz oder teilweise in Salzform vorliegen.

Bevorzugt sind die Alkali-, Erdalkali-, Ammonium- und Aminsalze. Besonders bevorzugt sind die Natrium- und Ammoniumsalze.

Die genannten vernetzenden Strukturen können sich von allen geeigneten Monomeren mit mindestens zwei olefinisch ungesättigten Doppelbindungen ableiten.

Geeignete Monomere sind beispielsweise Verbindungen, die mindestens zwei Alkenylgruppen, zum Beispiel Vinyl oder Allyl, oder mindestens zwei Alkenoylgruppen, zum Beispiel Acrylat oder Methacrylat, enthalten.

Bevorzugt leiten sich die vernetzenden Strukturen von Monomeren ab, die 2, 3 oder 4 ethylenisch ungesättigte Doppelbindungen enthalten.

Besonders bevorzugt leiten sich die vernetzenden Strukturen von Methyl-Bisacrylsäureamid oder N,N'-Dihydroxyethylen-Bisacrylsäureamid ab. Weiterhin können sich die vernetzenden Strukturen auch von kationischen Monomeren, wie beispielsweise Diallyl-Dimethyl-Ammoniumchlorid ableiten.

Die erfindungsgemäßen Polymerisate können hergestellt werden durch Polymerisation von 98 bis 100 Gew.%, bevorzugt 98,5 bis 99,7 Gew.%, einer Verbindung der allgemeinen Formel Ia

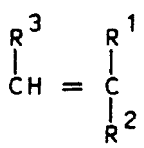

(Ia)

worin $R^1$, $R^2$ und $R^3$ wie oben definiert sind, oder eines Salzes davon mit 0 bis 2 Gew.%, bevorzugt 0,3 bis 1,5 Gew.%, eines Monomeren mit mindestens zwei olefinisch ungesättigten Doppelbindungen, dadurch gekennzeichnet, daß als Polymerisationsmedium eine wäßrige, ein Tensid und die Monomeren enthaltende, durch eine flüssige Kohlenwasserstoffphase stabilisierte Matrix eingesetzt wird.

Das Polymerisationsmedium besteht bevorzugt aus 50 bis 99,5 Gew.% Kohlenwasserstoff, 0,2 bis 20 Gew.% aus Monomer, 0,1 bis 5 Gew.% aus Tensid und 0,2 bis 25 Gew.% aus Wasser.

Besonders bevorzugt besteht das Polymerisationsmedium aus 60 bis 99 Gew.% aus Kohlenwasserstoff, 0,4 bis 16 Gew.% aus Monomer, 0,1 bis 4 Gew.% aus Tensid und 0,5 bis 20 Gew.% aus Wasser.

Das Polymerisationsmedium wird bevorzugterweise aufgebaut, indem man eine wäßrige, Tensid und Monomer enthaltende Lösung auf schäumt, wozu bevorzugt ein Inertgas, beispielsweise Stickstoff, verwendet wird, und dann den flüssigen Kohlenwasserstoff zugibt.

Dabei entsteht eine quasi-Schaumstruktur, wobei, verglichen mit einer herkömmlichen Schaumstruktur, die Kohlenwasserstoffphase die Stelle der Luft einnimmt. In Analogie zur Luft/Wasser-Schaumstruktur ist auch hier die wäßrige Phase die durchgehende, das heißt kontinuierliche Phase.

Als Tensid können alle gängigen Tenside anionischer, kationischer und nonionischer Struktur verwendet werden. Bevorzugt sind die Tenside nachstehender Strukturen:

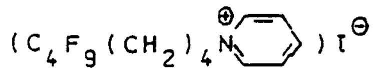

$C_5F_{11}COOH$

$C_6F_{13}CH_2COOH$

$C_8F_{17}COO^{\ominus}NH_4^{\oplus}$

$C_9F_{19}COOH$

$C_9F_{19}COO^{\ominus}NH^{\oplus}(CH_3)_3$

$C_9F_{19}COO^{\ominus}N^{\oplus}(CH_3)_4$

$C_9F_{19}COO^{\ominus}NH_4^{\oplus}$

$C_9F_{17}COO^{\ominus}NH_3^{\oplus}(C_2H_5)$

$C_9F_{17}COO^{\ominus}NH_3^{\oplus}CH_3$

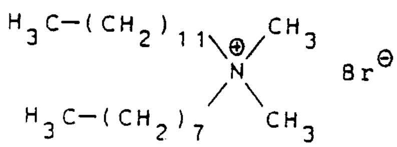

$(H_3C-(CH_2)_{11})_2-N^{\oplus}-(CH_3)_2Br^{\ominus}$

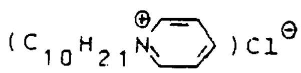

$C_{10}H_{21}SO_4^{\ominus}Na^{\oplus}$

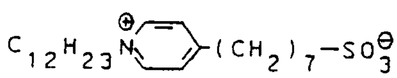

$C_{12}H_{25}SO_4^{\ominus}Na^{\oplus}$

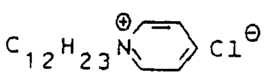

$C_{12}H_{23}N^{\oplus}H_3Cl^{\ominus}$

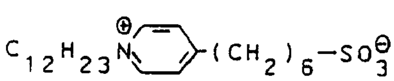

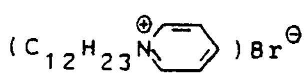

$C_{12}H_{23}N^{\oplus}(CH_3)_2-(CH_2)_8-SO_3^{\ominus}$

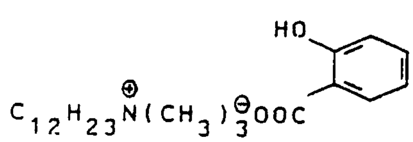

$C_{14}H_{29}N^{\oplus}(CH_3)_3Br^{\ominus}$

$C_{12}H_{23}N^{\oplus}(CH_3)_3Br^{\ominus}$

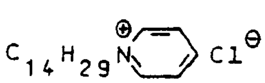

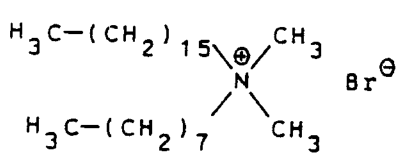

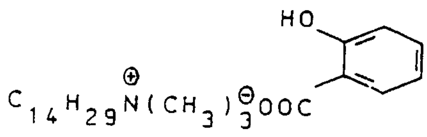

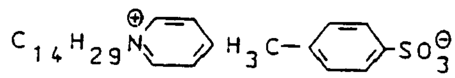

$C_{16}H_{33}\overset{\oplus}{N}(CH_3)_3Cl^{\ominus}$

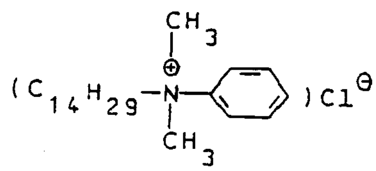

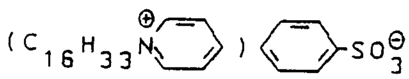

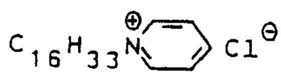

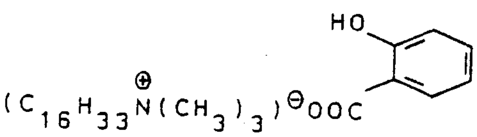

$(C_{16}H_{33}\overset{\oplus}{N}(CH_3)_3)_3PO_4^{-3}$

Ein ganz besonders bevorzugtes Tensid ist Dodecylsulfat-Natriumsalz.

Als Kohlenwasserstoffe können gesättigte sowie ungesättigte Aliphaten oder Aromaten verwendet werden. Sie können allein oder in Mischungen untereinander, wobei die Mischungsverhältnisse sind, eingesetzt werden.

Bevorzugte Kohlenwasserstoffe sind n-Pentan, n-Hexan, n-Heptan, n-Oktan, n-Nonan, n-Dekan, n-Dodekan, n-Tetradekan, n-Hexadekan, Cyclohexan, Cyclooktan, Benzol, Toluol, Kerosin, Benzin, bleifreies Benzin und Dieselöl.

Die Monomeren der allgemeinen Formel Ia sind bevorzugt wasserlösliche, olefinisch ungesättigte Verbindungen, wobei Säuren, wie beispielsweise Acrylsäure, Methacrylsäure, Vinylsulfonsäure, Styrolsulfonsäure, Maleinsäure, Fumarsäure, Crotonsäure, 2-Acrylamido-2-methyl-propansulfonsäure, 2-Acrylamido-2-methyl-propanphosphonsäure und Vinylphosphonsäure sowie deren Halbester, besonders bevorzugt sind. Geeignete wasserlösliche Monomere sind beispielsweise auch Acrylsäureamid, Methacrylsäureamid, Vinylpyrrolidon, Vinylpyridin, Vinylimidazolin sowie N-Vinylamide, wie beispielsweise N-Vinyl-N-Methyl-Acetamid, N-Vinyl-Formamid und N-Vinyl-Acetamid.

In bestimmten Grenzen können auch wasserunlösliche Monomere als Comonomere zugegeben werden. Beispiele sind die Alkylester der Acryl- und Methacrylsäure, Styrol, Vinylacetat, Acrylnitril und Vinylchlorid.

Besonders stabile erfindungsgemäße Polymerisate werden erhalten, wenn die Monomerenlösung Vernetzer, das heißt mehrfach olefinisch ungesättigte Verbindungen, die eine dreidimensionale Vernetzung der Polymerisate bewirken, enthält. Geeignete Monomere dieser Art sind beispielsweise solche mit mindestens zwei Alkenylgruppen, wie Vinyl oder Allyl, oder mindestens zwei Alkenoylgruppen, wie Acrylat oder Methacrylat.

Besonders bevorzugte Vernetzer sind Methyl-Bisacrylsäureamid und N,N'-Dihydroxyethylen-Bisacrylsäureamid.

Die erfindungsgemäßen Polymerisate können im erfindungsgemäßen Verfahren durch bekannte Polymerisationsreaktionen hergestellt werden. Bevorzugt wird eine Polymerisation wasserlöslicher Monomere in wässriger Lösung durchgeführt. Die relativ rasch verlaufende Polymerisation wird durch den Norrish-Trommsdorff-Effekt noch weiter beschleunigt.

Die Polymerisationsreaktion kann im Temperaturbereich zwischen 0°C und 130°C, vorzugsweise zwischen 10°C und 100°C, sowohl bei Normaldruck als auch unter erhöhtem Druck durchgeführt werden. Wie üblich kann die Polymerisation auch in einer Schutzgasatmosphäre, vorzugsweise unter Stickstoff, ausgeführt werden.

Zur Auslösung der Polymerisation können energiereiche elektromagnetische Strahlen oder die üblichen chemischen Polymerisationsinitiatoren herangezogen werden, z.B. organische Peroxide, wie Benzoylperoxid, tert. Butylhydroperoxid, Methyl-ethyl-keton-peroxid, Cumol-hydroperoxid, Azoverbindungen wie Azo-di-iso-butyro-nitril sowie anorganische Peroxiverbindungen wie $(NH_4)_2S_2O_8$ oder $K_2S_2O_8$ oder $H_2O_2$ gegebenenfalls in Kombination mit Reduktionsmitteln wie Natriumhydrogensulfit, und Eisen-II-Sulfat oder Redoxsysteme, welche als reduzierende Komponente eine aliphatische und aromatische Sulfinsäure, wie Benzolsulfinsäure und Toluolsulfinsäure oder Derivate dieser Säuren, enthalten, wie z.B. Mannichaddukte aus Sulfinsäure, Aldehyden und Aminoverbindungen, wie sie in der deutschen Patentschrift 1301566 beschrieben sind. Pro 100 g Gesamtmonomeren werden in der Regel 0,03 bis 2 g des Polymerisationsinitiators eingesetzt.

Das erfindungsgemäße Verfahren liefert nach Entfernen von Lösungsmittel, Kohlenwasserstoff und Tensid hochsaugfähige, poröse Polymerisatstrukturen, die sich hervorragend als Absorptionsmittel für Wasser und wäßrige Lösungen eignen, so daß sie vorteilhaft als wasserzurückhaltendes Mittel im landwirtschaftlichen Gartenbau, als Filtrationshilfsmittel und besonders als saugfähige Komponente in Hygieneartikeln wie Winde In, Tampons und Damenbinden eingesetzt werden können.

Beispiel 1

Eine Lösung von 2 g Acrylsäure, 2 g Dodecylsulfat-Natriumsalz und 0,1 g N,N'-(1,2-Dihydroxyethylen)-bis-acrylsäureamid werden in 4,9 g destilliertem Wasser gelöst und zusammen mit 40 g Heptan vorgelegt.

Zur Entfernung des in der Lösung befindlichen Sauerstoffs wird über einen Zeitraum von fünf Minuten mit Hilfe einer Glaskapillare Stickstoff durch das System geleitet. Nun wird das Gefäß mit einem Stopfen verschlossen und durch Schüttelbewegungen das dünnflüssige zweiphasige System in einen gelartigen Zustand überführt. Die Gelbildung ist beendet, wenn die gesamte Heptanmenge in die Gelphase eingearbeitet ist. Nun werden 0,018 g Kalium-Peroxodisulfat in 0,882 g Wasser, sowie 200 $\mu$l 10%ige Ascorbinsäurelösung zugegeben und in die Gelsubstanz eingeschüttelt. Auf diese Weise wird ein Polymerisationsstart innerhalb von 60 Minuten erreicht. Der Polymerisationsbeginn ist an einem Temperaturanstieg von etwa 15°C deutlich erkennbar. Etwa 10 bis 15 Minuten nach Polymerisationsbeginn ist die Reaktion beendet.

Der Polymerkörper wird nun in eine Schale überführt und etwa 8 Stunden mit einer Lösung von 1,4 g $NaHCO_3$ in einer Wassermenge, die gerade ausreicht, das Polymerisat vollständig zu bedecken, behandelt. In dieser Zeit wächst die Größe der mit Kohlenwasserstoff gefüllten Zellen von etwa 20-40 $\mu$m Durchmesser bis auf etwa 0,8 bis 1,2 mm an. Nun wird das sehr weiche und labile Produkt mit flüssigem Stickstoff übergossen und 30 Minuten im Mikrowellenherd gefriergetrocknet (alternativ kann dies auch im Gefriertrockner oder Rotationsverdampfer geschehen. Zeitbedarf hier etwa 8 Std.).

Das entstandene Produkt zeichnet sich durch ein schwammartiges Strukturbild aus, das von einem offenen, dreidimensionalen Kanalsystem durchsetzt ist. Die Porenweite beträgt 0,8 bis 1,2 mm. Die Dichte liegt im Bereich von 0,01 bis 0,05 g/cm$^3$.

Analog Beispiel 1 werden die folgenden Tabellenbeispiele 2 bis 36 hergestellt. In der Tabelle bedeuten die Mengenangaben Gew.% bezogen auf die vorgelegte Gesamtmenge.

Folgende Abkürzungen werden benutzt:

| | |
|---|---|
| AS | Acrylsäure |
| AMP | 2-Acrylamid-2-Methyl-Propansulfonsäure |
| AAM | Acrylsäureamid |
| ASME | Acrylsäuremethylester |
| TAE | Tetraallyloxyethan |
| DHEBA | N,N'-Dihydroxyethylen-Bisacrylsäureamid |
| MBA | Methyl-Bisacrylsäureamid |
| KPS | $K_2S_2O_8$ |
| $H_2O_2$ | Wasserstoffperoxid |
| APS | $(NH_4)_2S_2O_8$ |

Tabelle

| Beispiel | Monomer | Comonomer | Vernetzer | Starter | Kohlenwasserstoff | Tensid Gew.% | Monomer Gew.% | Comonomer Gew.% | Vernetzer Gew.% | Starter Gew.% | $H_2O$ Gew.% | Kohlenwasserstoff Gew.% |
|---|---|---|---|---|---|---|---|---|---|---|---|---|
| 2 | AS | | TAE | KPS | Pentan | 1,5 | 9 | | 0,45 | 0,09 | 18,96 | 70 |
| 3 | AS | | TAE | KPS | Hexan | 1 | 6 | | 0,3 | 0,06 | 12,64 | 80 |
| 4 | AS | | TAE | KPS | Heptan | 0,5 | 3 | | 0,15 | 0,03 | 6,05 | 90 |
| 5 | AS | | TAE | KPS | Oktan | 0,25 | 1,5 | | 0,075 | 0,015 | 3,16 | 95 |
| 6 | AS | | TAE | KPS | Dieselöl | 0,1 | 0,6 | | 0,03 | 0,006 | 1,26 | 98 |
| 7 | AS | | DHEBA | APS | Heptan | 1,5 | 6 | | 0,45 | 0,09 | 21,96 | 70 |
| 8 | AS | | DHEBA | APS | Dekan | 1 | 4 | | 0,3 | 0,06 | 14,64 | 80 |
| 9 | AS | | DHEBA | APS | Pentan | 0,5 | 2 | | 0,15 | 0,03 | 7,05 | 90 |
| 10 | AS | | DHEBA | APS | Hexan | 0,25 | 1 | | 0,075 | 0,015 | 3,66 | 95 |
| 11 | AS | | DHEBA | APS | Nonan | 0,1 | 0,4 | | 0,03 | 0,006 | 1,464 | 98 |
| 12 | AAM | | DHEBA | KPS | Pentan | 1,5 | 12 | | 0,45 | 0,09 | 15,96 | 70 |
| 13 | AAM | | DHEBA | KPS | Hexan | 1 | 8 | | 0,3 | 0,06 | 10,64 | 80 |
| 14 | AAM | | DHEBA | KPS | Heptan | 0,5 | 4 | | 0,15 | 0,03 | 5,05 | 90 |
| 15 | AAM | | DHEBA | KPS | Dodekan | 0,25 | 2 | | 0,075 | 0,015 | 2,66 | 95 |
| 16 | AAM | | DHEBA | KPS | Oktan | 0,1 | 0,8 | | 0,03 | 0,006 | 1,064 | 98 |
| 17 | AMP | | MBA | KPS | Pentan | 6 | 9 | | 0,3 | 0,06 | 14,64 | 70 |
| 18 | AMP | | MBA | KPS | Hexan | 4 | 6 | | 0,2 | 0,04 | 9,76 | 80 |
| 19 | AMP | | MBA | KPS | Heptan | 2 | 3 | | 0,1 | 0,02 | 4,88 | 90 |
| 20 | AMP | | MBA | KPS | Dekan | 1 | 1,5 | | 0,05 | 0,01 | 2,44 | 95 |
| 21 | AMP | | MBA | KPS | Dieselöl | 0,4 | 0,6 | | 0,02 | 0,004 | 0,976 | 98 |

Tabelle

| Beispiel | Monomer | Comonomer | Vernetzer | Starter | Kohlenwasserstoff | Tensid Gew.% | Monomer Gew.% | Comonomer Gew.% | Vernetzer Gew.% | Starter Gew.% | $H_2O$ Gew.% | Kohlenwasserstoff Gew.% |
|---|---|---|---|---|---|---|---|---|---|---|---|---|
| 22 | AS | ASME | DHEBA | KPS | Pentan | 6 | 3 | 3 | 0,3 | 0,06 | 17,64 | 70 |
| 23 | AS | ASME | DHEBA | KPS | Hexan | 4 | 1 | 3 | 0,2 | 0,04 | 11,76 | 80 |
| 24 | AS | ASME | DHEBA | KPS | Heptan | 2 | 1,5 | 0,5 | 0,1 | 0,02 | 5,88 | 90 |
| 25 | AS | ASME | DHEBA | KPS | Oktan | 1 | 0,5 | 0,5 | 0,05 | 0,01 | 2,94 | 95 |
| 26 | AS | ASME | DHEBA | KPS | Nonan | 0,4 | 0,1 | 0,3 | 0,02 | 0,004 | 1,176 | 98 |
| 27 | AAM | | TAE | $H_2O_2$ | Pentan | 6 | 6 | | 0,3 | 0,06 | 17,64 | 70 |
| 28 | AAM | | TAE | $H_2O_2$ | Hexan | 4 | 4 | | 0,2 | 0,04 | 11,76 | 80 |
| 29 | AAM | | TAE | $H_2O_2$ | Heptan | 2 | 2 | | 0,1 | 0,02 | 5,88 | 90 |
| 30 | AAM | | TAE | $H_2O_2$ | Oktan | 1 | 1 | | 0,05 | 0,01 | 2,94 | 95 |
| 31 | AAM | | TAE | $H_2O_2$ | Nonan | 0,4 | 0,4 | | 0,02 | 0,004 | 1,176 | 98 |
| 32 | AS | | DHEBA | APS | Dekan | 6 | 12 | | 0,3 | 0,06 | 11,64 | 70 |
| 33 | AS | | DHEBA | APS | Cyclohexan | 4 | 8 | | 0,2 | 0,04 | 7,76 | 80 |
| 34 | AS | | DHEBA | APS | Benzol | 2 | 4 | | 0,1 | 0,02 | 3,88 | 90 |
| 35 | AS | | DHEBA | APS | Toluol | 1 | 2 | | 0,05 | 0,01 | 1,94 | 95 |
| 36 | AS | | DHEBA | APS | Dieselöl | 0,4 | 0,8 | | 0,02 | 0,004 | 0,776 | 98 |

## Patentansprüche

## Patentansprüche für folgende Vertragsstaaten : AT, BE, CH, DE, DK, FR, GB, IT, LI, NL, SE

1. Hydrophiles, quellbares Polymerisat, das in statistischer Verteilung zu 98 bis 100 Gew.% aus Resten der allgemeinen Formel I

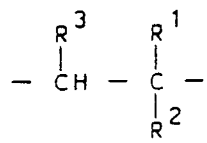

(I)

worin

$R^1$ Wasserstoff, Methyl oder Ethyl

$R^2$ Carboxyl, Sulfonyl, Phosphonyl, die gegebenenfalls mit Alkanol mit 1 bis 4 Kohlenstoffatomen verestert sein können; Phenyl; Sulfonylphenyl; Pyrrolidonyl; Pyridyl; Imidazolyl; eine Gruppe der Formel

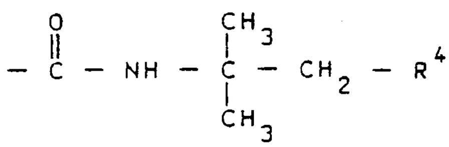

worin $R^4$ für die Sulfonyl- oder die Phosphonylgruppe steht; Cyan; Chlor; die $-CONH_2$-Gruppe; $(C_1-C_4)$-Alkanoyloxy; oder eine Gruppe der Formel

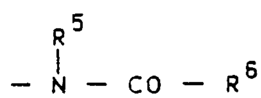

worin $R^5$ und $R^6$ unabhängig voneinander Wasserstoff, Methyl oder Ethyl bedeuten oder gegebenenfalls auch zusammen für Trimethylen stehen und

$R^3$ Wasserstoff, Methyl, Ethyl oder Carboxy bedeuten, wobei saure Gruppen auch in Salzform vorliegen können, und zu 0 - 2 Gew.% aus Resten eines Vernetzers, die aus Monomeren mit mindestens zwei olefinisch ungesättigten Doppelbindungen hervorgegangen sind, besteht, dadurch gekennzeichnet, daß es in Form einer hochporösen, schaumartigen Polyederstruktur mit einem mittleren Porendurchmesser von 0.8 bis 1.2 mm vorliegt.

2. Polymerisat nach Anspruch 1, dadurch gekennzeichnet, daß es zu 98,5 bis 99,7 Gew.% aus Resten der allgemeinen Formel I und zu 0,3 bis 1,5 Gew.% aus vernetzenden Strukturen besteht.

3. Polymerisat nach Anspruch 1 und/oder 2, dadurch gekennzeichnet, daß in der allgemeinen Formel I $R^1$ Wasserstoff oder Methyl, $R^2$ Carboxyl, Sulfonyl oder Phosphonyl, besonders bevorzugt Carboxyl und $R^3$ Wasserstoff bedeutet.

4. Polymerisat nach einem oder mehreren der Ansprüche 1 bis 3, dadurch gekennzeichnet, daß sich die Reste eines Vernetzers von Methyl-Bisacrylsäureamid oder N,N'-Dihydroxyethylen-Bisacrylsäureamid ableiten.

5. Verfahren zur Herstellung hydrophiler, quellbarer Polymerisate gemäß einem der Ansprüche 1 bis 4 durch Polymerisation von 98 bis 100 Gew.%, bevorzugt 98,5 bis 99,7 Gew.%, einer Verbindung der allgemeinen Formel Ia

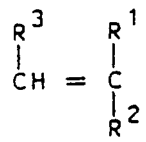

(Ia)

worin $R^1$, $R^2$ und $R^3$ wie oben definiert sind, oder eines Salzes davon mit 0 bis 2 Gew.%, bevorzugt 0,3 bis 1,5 Gew.%, eines Monomeren mit mindestens zwei olefinisch ungesättigten Doppelbindungen, dadurch gekennzeichnet, daß als Polymerisationsmedium eine wäßrige, ein Tensid und die Monomeren enthaltende, durch eine flüssige Kohlenwasserstoffphase stabilisierte Matrix eingesetzt wird.

**6.** Verfahren nach Anspruch 5, dadurch gekennzeichnet, daß das Polymerisationsmedium aus 50 bis 99,5 Gew.%, besonders bevorzugt 60 bis 99 Gew.%, Kohlenwasserstoff, 0,2 bis 20 Gew.%, besonders bevorzugt 0,4 bis 16 Gew.%, Monomer, 0,1 bis 5 Gew.%, besonders bevorzugt 0,1 bis 4 Gew.%, Tensid und 0,2 bis 25 Gew.%, besonders bevorzugt 0,5 bis 20 Gew.%, aus Wasser besteht.

**7.** Verfahren nach Anspruch 5 und/oder 6, dadurch gekennzeichnet, daß als Kohlenwasserstoff n-Pentan, n-Hexan, n-Heptan, n-Oktan, n-Nonan, n-Dekan, n-Dodekan, n-Tetradekan, n-Hexadekan, Cyclohexan, Cyclooktan, Benzol, Toluol, Kerosin, Benzin, bleifreies Benzin oder Dieselöl eingesetzt wird.

**8.** Verfahren nach einem oder mehreren der Ansprüche 5 bis 7, dadurch gekennzeichnet, daß als Tensid Dodecylsulfat-Natriumsalz eingesetzt wird.

**9.** Verwendung der Polymerisate gemäß den Ansprüchen 1 bis 4 als saugfähige Komponente in Hygieneartikeln, wie Windeln, Tampons und Damenbinden.

**Patentansprüche für folgenden Vertragsstaat : ES**

**1.** Verfahren zur Herstellung eines hydrophilen, quellbaren Polymerisats, das in statistischer Verteilung zu 98 bis 100 Gew.% aus Resten der allgemeinen Formel I

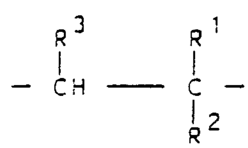

(I)

worin

$R^1$ Wasserstoff, Methyl oder Ethyl

$R^2$ Carboxyl, Sulfonyl, Phosphonyl, die gegebenenfalls mit Alkanol mit 1 bis 4 Kohlenstoffatomen verestert sein können; Phenyl; Sulfonylphenyl; Pyrrolidonyl; Pyridyl; Imidazolyl; eine Gruppe der Formel

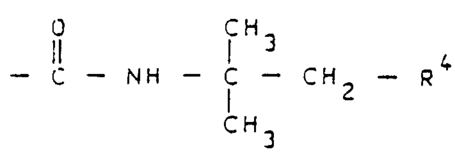

worin $R^4$ für die Sulfonyl- oder die Phosphonylgruppe steht; Cyan; Chlor; die $-CONH_2$-Gruppe; $(C_1-C_4)$-Alkanoyloxy; oder eine Gruppe der Formel

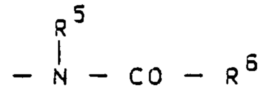

worin $R^5$ und $R^6$ unabhängig voneinander Wasserstoff, Methyl oder Ethyl bedeuten oder gegebenenfalls auch zusammen für Trimethylen stehen und
$R^3$ Wasserstoff, Methyl, Ethyl oder Carboxy bedeuten, wobei saure Gruppen auch in Salzform vorliegen können und
zu 0 - 2 Gew.% aus Resten eines Vernetzers, die aus Monomeren mit mindestens zwei olefinisch ungesättigten Doppelbindungen hervorgegangen sind, besteht, durch Polymerisation von 98 bis 100 Gew.% einer Verbindung der allgemeinen Formel Ia

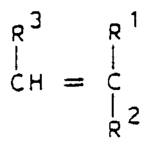

(Ia)

worin $R^1$, $R^2$ und $R^3$ wie oben definiert sind, oder eines Salzes davon mit 0 bis 2 Gew.% eines Monomeren mit mindestens zwei olefinisch ungesättigten Doppelbindungen, dadurch gekennzeichnet, daß als Polymerisationsmedium eine wäßrige, ein Tensid und die Monomeren enthaltende, durch eine flüssige Kohlenwasserstoffphase stabilisierte Matrix eingesetzt wird.

**2.** Verfahren nach Anspruch 1, dadurch gekennzeichnet, daß das Polymerisationsmedium aus 50 bis 99,5 Gew.%, besonders bevorzugt 60 bis 99 Gew.%, Kohlenwasserstoff, 0,2 bis 20 Gew.%, besonders bevorzugt 0,4 bis 16 Gew.%, Monomer, 0,1 bis 5 Gew.%, besonders bevorzugt 0,1 bis 4 Gew.%, Tensid und 0,2 bis 25 Gew.%, besonders bevorzugt 0,5 bis 20 Gew.%, aus Wasser besteht.

**3.** Verfahren nach Anspruch 1 und/oder 2, dadurch gekennzeichnet, daß als Kohlenwasserstoff n-Pentan, n-Hexan, n-Heptan, n-Oktan, n-Nonan, n-Dekan, n-Dodekan, n-Tetradekan, n-Hexadekan, Cyclohexan, Cyclooktan, Benzol, Toluol, Kerosin, Benzin, bleifreies Benzin oder Dieselöl eingesetzt wird.

**4.** Verfahren nach einem oder mehreren der Ansprüche 1 bis 3, dadurch gekennzeichnet, daß als Tensid Dodecylsulfat-Natriumsalz eingesetzt wird.

**5.** Verfahren nach einem oder mehreren der Ansprüche 1 bis 4, dadurch gekennzeichnet, daß das hergestellte Polymerisat eine Polyederstruktur mit einem mittleren Porendurchmesser von 0,8 bis 1,2 mm aufweist.

**6.** Verfahren nach einem oder mehreren der Ansprüche 1 bis 5, dadurch gekennzeichnet, daß das hergestellte Polymerisat zu 98,5 bis 99,7 Gew.% aus Resten der allgemeinen Formel I und zu 0,3 bis 1,5 Gew.% aus venetzenden Strukturen besteht.

**7.** Verfahren nach einem oder mehreren der Ansprüche 1 bis 6, dadurch gekennzeichnet, daß in der allgemeinen Formel I $R^1$ Wasserstoff oder Methyl, $R^2$ Carboxyl, Sulfonyl oder Phosphonyl, besonders bevorzugt Carboxyl und $R^3$ Wasserstoff bedeutet.

**8.** Verfahren nach einem oder mehreren der Ansprüche 1 bis 7, dadurch gekennzeichnet, daß sich die Reste eines Vernetzers von Methyl-Bisacrylsäureamid oder N,N′-Dihydroxyethylen-Bisacrylsäureamid ableiten.

**9.** Verwendung der nach einem oder mehreren der Ansprüche 1 bis 8 hergestellten Polymerisate als saugfähige Komponente in Hygieneartikeln, wie Windeln, Tampons und Damenbinden.

**Claims**

**Claims for the following Contracting States : AT, BE, CH, DE, DK, FR, GB, IT, LI, NL, SE**

**1.** Hydrophilic, swellable polymer which consists, in random distribution, to the extent of 98 to 100% by weight of radicals of the general formula I

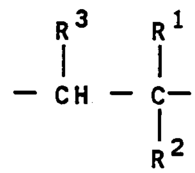

(I)

wherein
$R^1$ denotes hydrogen, methyl or ethyl,
$R^2$ denotes carboxyl, sulphonyl or phosphonyl, which can optionally be esterified by alkanol having 1 to 4 carbon atoms; phenyl; sulphonylphenyl; pyrrolidonyl; pyridyl; imidazolyl; a group of the formula

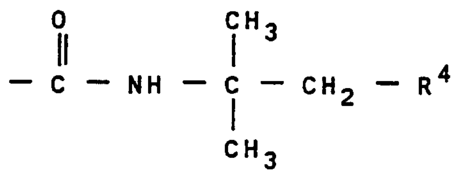

wherein $R^4$ represents the sulphonyl or the phosphonyl group; cyano; chlorine; the $-CONH_2$ group; ($C_1$-$C_4$)-alkanoyloxy; or a group of the formula

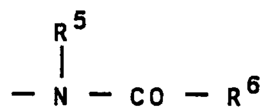

wherein $R^5$ and $R^6$ independently of one another denote hydrogen, methyl or ethyl or optionally also together represent trimethylene; and
$R^3$ denotes hydrogen, methyl, ethyl or carboxyl, it also being possible for acid groups to be present in salt form, and to the extent of 0 - 2% by weight of radicals of a crosslinking agent which have originated from monomers having at least two olefinically unsaturated double bonds, characterized in that it is in the form of a highly porous, foam-like polyhedral structure having an average pore diameter of 0.8 to 1.2 mm.

**2.** Polymer according to Claim 1, characterized in that it consists to the extent of 98.5 to 99.7% by weight of radicals of the general formula I and to the extent of 0.3 to 1.5% by weight of crosslinking structures.

**3.** Polymer according to Claim 1 and/or 2, characterized in that in the general formula I, $R^1$ denotes hydrogen or methyl, $R^2$ denotes carboxyl, sulphonyl or phosphonyl, particularly preferably carboxyl, and $R^3$ denotes hydrogen.

**4.** Polymer according to one or more of Claims 1 to 3, characterized in that the radicals of a crosslinking agent are derived from methyl-bisacrylamide or N,N'-dihydroxyethylenebisacrylamide.

**5.** Process for the preparation of hydrophilic, swellable polymers according to one of Claims 1 to 4 by polymerization of 98 to 100% by weight, preferably 98.5 to 99.7% by weight, of a compound of the general formula Ia

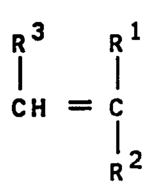

(Ia)

wherein $R^1$, $R^2$ and $R^3$ are as defined above, or a salt thereof, with 0 to 2% by weight, preferably 0.3 to 1.5% by weight, of a monomer having at least two olefinically unsaturated double bonds, characterized in that an aqueous matrix containing a surfactant and the monomers and stabilized by a liquid hydrocarbon phase is employed as the polymerization medium.

6. Process according to Claim 5, characterized in that the polymerization medium consists of 50 to 99.5% by weight, particularly preferably 60 to 99% by weight, of hydrocarbon, 0.2 to 20% by weight, particularly preferably 0.4 to 16% by weight, of monomer, 0.1 to 5% by weight, particularly preferably 0.1 to 4% by weight, of surfactant and 0.2 to 25% by weight, particularly preferably 0.5 to 20% by weight, of water.

7. Process according to Claim 5 and/or 6, characterized in that n-pentane, n-hexane, n-heptane, n-octane, n-nonane, n-decane, n-dodecane, n-tetradecane, n-hexadecane, cyclohexane, cyclooctane, benzene, toluene, kerosine, petrol, lead-free petrol or diesel oil is employed as the hydrocarbon.

8. Process according to one or more of Claims 5 to 7, characterized in that sodium dodecylsulphate is employed as the surfactant.

9. Use of the polymers according to Claim 1 to 4 as absorbent components in hygiene articles, such as nappies, tampons and sanitary towels.

**Claims for the following Contracting State : ES**

1. Process for preparing a hydrophilic, swellable polymer which consists, in random distribution, to the extent of 98 to 100% by weight of radicals of the general formula I

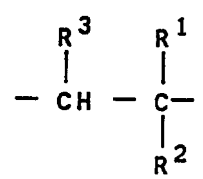

(I)

wherein
$R^1$ denotes hydrogen, methyl or ethyl,
$R^2$ denotes carboxyl, sulphonyl or phosphonyl, which can optionally be esterified by alkanol having 1 to 4 carbon atoms; phenyl; sulphonylphenyl; pyrrolidonyl; pyridyl; imidazolyl; a group of the formula

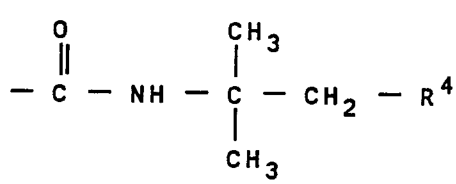

wherein $R^4$ represents the sulphonyl or the phosphonyl group; cyano; chlorine; the $-CONH_2$ group; $(C_1-C_4)$-alkanoyloxy; or a group of the formula

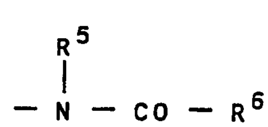

wherein $R^5$ and $R^6$ independently of one another denote hydrogen, methyl or ethyl or optionally also together represent trimethylene; and

$R^3$ denotes hydrogen, methyl, ethyl or carboxyl, it also being possible for acid groups to be present in salt form, and to the extent of 0 - 2% by weight of radicals of a crosslinking agent which have originated from monomers having at least two olefinically unsaturated double bonds by polymerization of 98 to 100% by weight of a compound of the general formula Ia

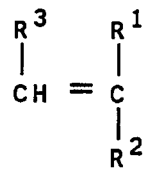

(Ia)

wherein $R^1$, $R^2$ and $R^3$ are as defined above, or a salt thereof, with 0 to 2% by weight of a monomer having at least two olefinically unsaturated double bonds, characterized in that an aqueous matrix containing a surfactant and the monomers and stabilized by a liquid hydrocarbon phase is employed as the polymerization medium.

**2.** Process according to Claim 1, characterized in that the polymerization medium consists of 50 to 99.5% by weight, particularly preferably 60 to 99% by weight, of hydrocarbon, 0.2 to 20% by weight, particularly preferably 0.4 to 16% by weight, of monomer, 0.1 to 5% by weight, particularly preferably 0.1 to 4% by weight, of surfactant and 0.2 to 25% by weight, particularly preferably 0.5 to 20% by weight, of water.

**3.** Process according to Claim 1 and/or 2, characterized in that n-pentane, n-hexane, n-heptane, n-octane, n-nonane, n-decane, n-dodecane, n-tetradecane, n-hexadecane, cyclohexane, cyclooctane, benzene, toluene, kerosine, petrol, lead-free petrol or diesel oil is employed as the hydrocarbon.

**4.** Process according to one or more of Claims 1 to 3, characterized in that sodium dodecylsulphate is employed as the surfactant.

**5.** Process according one or more of Claims 1 to 4, characterized in that the produced polymer has a polyhedral structure having an average pore diameter of 0.8 to 1.2 mm.

**6.** Process according to one or more of Claims 1 to 5, characterized in that the produced polymer consists to the extent of 98.5 to 99.7% by weight of radicals of the general formula I and to the extent of 0.3 to 1.5% by weight of crosslinking structures.

**7.** Process according to one or more of Claims 1 to 6, characterized in that in the general formula I, $R^1$ denotes hydrogen or methyl, $R^2$ denotes carboxyl, sulphonyl or phosphonyl, particularly preferably carboxyl, and $R^3$ denotes hydrogen.

**8.** Process according to one or more of Claims 1 to 7, characterized in that the radicals of a crosslinking agent are derived from methyl-bisacrylamide or N,N'-dihydroxyethylenebisacrylamide.

**9.** Use of the polymers produced according to one or more of Claims 1 to 8 as absorbent components in hygiene articles, such as nappies, tampons and sanitary towels.

**Revendications**

**Revendications pour les Etats contractants suivants : AT, BE, CH, DE, DR, FR, GB, IT, LI, NL, SE**

**1.** Polymère gonflable hydrophile qui se compose en une répartition statistique de 98 à 100% en poids de radicaux formule générale I

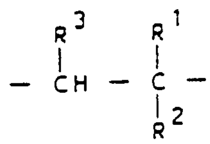

( I )

où

$R^1$ est l'hydrogène, un méthyle ou un éthyle,

$R^2$ est un carboxyle, un sulfonyle, un phosphonyle qui peuvent éventuellement être estérifiés avec un alcanol comportant 1 à 4 atomes de carbone ; un phényle ; un sulfonylphényle ; un pyrrolidonyle ; un pyridyle ; un imidazolyle ; un groupe de formule

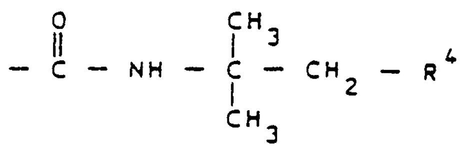

où R4 représente le groupe sulfonyle ou phosphonyle ; un groupe cyano ; le chlore ; le groupe $-CONH_2$ ; un alcanoyloxy en $C_1$-$C_4$ ou un groupe de formule

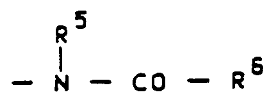

dans laquelle $R^5$ et $R^6$ indépendamment l'un de l'autre signifient l'hydrogène, un méthyle ou un éthyle ou, éventuellement ensemble, aussi le triméthylène ; et

$R^3$ signifie l'hydrogène, un méthyle, un éthyle ou un carboxy, les groupes acides pouvant être présents aussi sous forme de sels, et de 0 à 2% en poids de radicaux d'un agent réticulant, qui proviennent de monomères avec au moins deux doubles liaisons à insaturation oléfinique, caractérisé en ce qu'il est présent sous forme d'une structure polyédrique expansée, très poreuse, avec un diamètre moyen de pores de 0,8 à 1,2 mm.

**2.** Polymère selon la revendication 1, caractérisé en ce qu'il se compose de 98,5 à 99,7% en poids de radicaux de formule générale I et de 0,3 à 1,5% en poids de structures réticulantes.

**3.** Polymère selon la revendication 1 et/ou 2, caractérisé en ce que dans la formule générale I, $R^1$ signifie l'hydrogène ou un méthyle, $R^2$ un carboxyle, un sulfonyle ou un phosphonyle, de manière particulièrement préférée un carbonyle, et $R^3$ signifie l'hydrogène.

**4.** Polymère selon l'une ou plusieurs revendications 1 à 3, caractérisé en ce que les radicaux d'un agent réticulant dérivent du méthyl-bisacrylamide ou de N,N'-dihydroxyéthylène-bisacrylamide.

**5.** Procédé pour la préparation de polymères gonflabes, hydrophiles, selon l'une des revendications 1 à 4 par polymérisation de 98 à 100% en poids, de préférence de 98,5 à 99,7% en poids, d'un composé de formule générale Ia

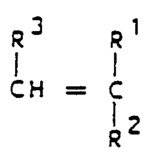

( I a )

où $R^1$, $R^2$ et $R^3$ sont définis comme ci-dessus, ou d'un de ses sels avec 0 à 2% en poids, de préférence de 0,3 à 1,5% en poids, d'un monomère comportant au moins deux doubles liaisons à insaturation oléfinique, caractérisé en ce qu'on utilise en tant que milieu de polymérisation une matrice aqueuse, contenant un agent de surface et les monomères, stabilisée par une phase hydrocarbonée liquide.

6. Procédé selon la revendication 5, caractérisé en ce que le milieu de polymérisation se compose de 50 à 99,5% en poids, de préférence plus particulièrement de 60 à 99% en poids d'hydrocarbone, de 0,2 à 20% en poids, de préférence plus particulièrement de 0,4 à 16% en poids, de monomère, de 0,1 à 5% en poids de préférence de 0,1 à 4% en poids d'agent de surface et de 0,2 à 25% en poids, de préférence plus particulièrement de 0,5 à 20% en poids d'eau.

7. Procédé selon la revendication 5 et/ou 6, caractérisé en ce qu'on utilise en tant que phase hydrocarbonée le n-pentane, le n-hexane, le n-heptane, le n-octane, le n-nonane, le n-décane, le n-dodécane, le n-tétradécane, le n-hexadécane, le cyclohexane, le cyclooctane, le benzène, le toluène, le kérosène, l'essence, l'essence sans plomb et le carburant diesel.

8. Procédé selon une ou plusieurs revendications 5 à 7, caractérisé en ce qu'on utilise en tant qu'agent de surface le sel de sodium du dodécylsulfate.

9. Utilisation des polymères selon les revendications 1 à 4 en tant que composants à pouvoir absorbant dans des articles d'hygiène, comme les couches, les tampons et les serviettes périodiques.

**Revendications pour l'Etat contractant suivant : ES**

1. Procédé pour la préparation d'un polymère gonflable, hydrophile, qui se compose en une répartition statistique de 98 à 100% en poids de radicaux de formule générale I

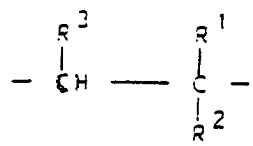

( I )

où
$R^1$ est l'hydrogène, un méthyle ou un éthyle,
$R^2$ est un carboxyle, un sulfonyle, un phosphonyle qui peuvent éventuellement être estérifiés avec un alcanol comportant 1 à 4 atomes de carbone ; un phényle ; un sulfonylphényle ; un pyrrolidonyle ; un pyridyle ; un imidazolyle ; un groupe de formule

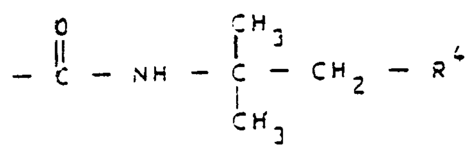

où R4 représente le groupe sulfonyle ou phosphonyle ; un groupe cyano ; le chlore ; le groupe $-CONH_2$ ; un alcanoyloxy en $C_1$-$C_4$ ou un groupe de formule

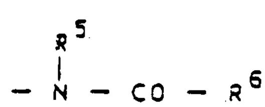

dans laquelle $R^5$ et $R^6$ indépendamment l'un de l'autre représentent l'hydrogéne, un méthyle ou un

éthyle ou, éventuellement ensemble, aussi le triméthylène ; et
$R^3$ signifie l'hydrogène, un méthyle, un éthyle ou un carboxy, les groupes acides pouvant être présents aussi sous forme de sels, et se composent de 0 à 2% en poids de radicaux d'un agent réticulant qui proviennent de monomères comportant au moins deux doubles liaisons à insaturation oléfinique, par polymérisation de 98 à 100% en poids d'un composé de formule générale Ia

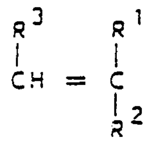

(Ia)

où $R^1$, $R^2$ et $R^3$ sont définis comme ci-dessus, ou d'un de leurs sels avec 0 à 2% en poids d'un monomère avec au moins deux doubles liaisons à insaturation oléfinique, caractérisé en ce qu'on utilise en tant que milieu de polymérisation une matrice aqueuse, contenant un agent de surface et les monomères, stabilisée par une phase hydrocarbonée liquide.

**2.** Procédé selon la revendication 1, caractérisé en ce que le milieu de polymérisation se compose de 50 à 99,5% en poids, de préférence plus particulièrement de 60 à 99% en poids d'hydrocarbone, de 0,2 à 20% en poids, de préférence plus particulièrement de 0,4 à 16% en poids, de monomère, de 0,1 à 5% en poids, de préférence plus particulièrement de 0,1 à 4% en poids, d'agent de surface, et de 0,2 à 25% en poids, de préférence plus particulièrement de 0,5 à 20% en poids d'eau.

**3.** Procédé selon la revendication 1 et/ou 2, caractérisé en ce qu'on utilise en tant qu'hydrocarbone le n-pentane, le n-hexane, le n-heptane, le n-octane, le n-nonane, le n-décane, le dodécane, le n-tétradécane, le n-hexadécane, le cyclohexane, le cyclooctane, le benzène, le toluène, le kérosène, l'essence, l'essence sans plomb et le carburant diesel.

**4.** Procédé selon l'une ou plusieurs revendications 1 à 3, caractérisé en ce qu'on utilise en tant qu'agent de surface le sel de sodium du dodécylsulfate.

**5.** Procédé selon l'une ou plusieurs revendications 1 à 4, caractérisé en ce que le polymère préparé présente une structure polyédrique avec un diamètre moyen de pores allant de 0,8 à 1,2 mm.

**6.** Procédé selon l'une ou plusieurs revendications 1 à 5, caractérisé en ce que le polymère préparé se composé de 98,5 à 99,7% en poids de radicaux de formule générale I et de 0,3 à 1,5% en poids de structures réticulantes.

**7.** Procédé selon l'une ou plusieurs revendications 1 à 6, caractérisé en ce que dans la formule générale I $R^1$ signifie l'hydrogène ou un éthyle, $R^2$ un carboxyle, un sulfonyle ou un phosphonyle, de préférence plus particulièrement un carboxyle, et $R^3$ l'hydrogène.

**8.** Procédé selon l'une ou plusieurs revendications 1 à 7, caractérisé en ce que les radicaux d'un agent réticulant proviennent du méthyl-bis-acrylamide ou du N,N'-dihydroxyéthylène-bis-acrylamide.

**9.** Utilisation du polymère préparé selon l'une ou plusieurs revendications 1 à 8, en tant que composant à pouvoir absorbant dans des articles d'hygiène comme les couches, les tampons et les serviettes périodiques.